# EUROPÄISCHE PATENTSCHRIFT

(11) **EP 0 944 585 B1**
(45) Veröffentlichungstag und Bekanntmachung des Hinweises auf die Patenterteilung: **17.04.2002**
(21) Anmeldenummer: 97954375.8
(22) Anmeldetag: 10.12.1997
(51) Int. Cl.: C07C 253/10, C07C 255/07, C07C 7/167, C07C 255/04

(54) **MONOOLEFINISCHE C 5-MONONITRILE, VERFAHREN ZU IHRER HERSTELLUNG UND IHRER VERWENDUNG**
MONOOLEFINIC C 5-MONONITRILES, METHOD FOR THE PRODUCTION AND THE USE THEREOF
MONONITRILES C 5 MONOOLEFINIQUES, LEUR PROCEDE DE PREPARATION ET LEUR UTILISATION

(30) Priorität: 16.12.1996 DE 19652273
(43) Veröffentlichungstag der Anmeldung: 29.09.1999
(73) Patentinhaber: BASF AKTIENGESELLSCHAFT, 67056 Ludwigshafen (DE)
(72) Erfinder: FISCHER, Jakob, D-85414 Kirchdorf (DE); SIEGEL, Wolfgang, D-67117 Limburgerhof (DE); MUNDINGER, Klaus, D-67117 Limburgerhof (DE); MEYER, Gerald, D-67071 Ludwigshafen (DE)
(74) Vertreter: Kinzebach, Werner, Dr.
(86) Internationale Anmeldenummer: EP9706900
(87) Internationale Veröffentlichungsnummer: WO9827054

(56) Entgegenhaltungen:
- EP-A- 0 273 900
- EP-A- 0 650 959
- WO-A-96/22968
- FR-A- 2 212 311
- GB-A- 1 161 645
- US-A- 3 900 526

## Beschreibung

Die vorliegende Erfindung betrifft monoolefinische C₅-Mononitrile, bei denen die C=C- und die C≡N-Bindung nicht konjugiert sind, ein Verfahren zu ihrer Herstellung durch katalytische Hydrocyanierung eines 1,3-Butadien-haltigen Kohlenwasserstoffgemisches und ihre Verwendung als Zwischenprodukte für die Weiterverarbeitung zu Adipodinitril.

Die Herstellung von Gemischen monoolefinischer C₅-Mononitrile, die z.B. 3-Pentennitril und 2-Methyl-3-butennitril enthalten, aus reinem 1,3-Butadien ist äußerst unwirtschaftlich, da dieses durch aufwendige extraktive Destillation aus technisch erhältlichen Kohlenwasserstoffgemischen isoliert werden muß.

1,3-Butadien-haltige Kohlenwasserstoffgemische sind in großtechnischem Maßstab erhältlich. So fällt z.B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im allgemeinen bis zu 5 % an Alkinen, 1,2-Dienen und Vinylacetylen, die nur durch aufwendige und somit teure verfahrenstechnische Maßnahmen abgetrennt werden können.

Eine Möglichkeit zur Entfernung von Acetylenen und Allenen aus Diolefin-haltigen Kohlenwasserstoffgemischen besteht in der Teilhydrierung dieser Gemische in Gegenwart von Katalysatoren, die befähigt sind, zwischen diesen Substanzklassen zu differenzieren. So beschreibt die US-A-4,587,369 selektive Hydrierkatalysatoren auf Basis von Pd auf einem Aluminiumoxid-Träger.

Die US-A-4,704,492 beschreibt Cu/Pd-Kontakte als selektive Hydrierkatalysatoren.

Bei bestimmten Verwendungen des Kohlenwasserstoffgemisches ist ein möglichst geringer Verlust an Diolefin, z.B. an 1,3-Butadien, bei gleichzeitig möglichst vollständiger Entfernung der Acetylene erwünscht. Die US-A-4,493,906 beschreibt einen Katalysator auf Basis von fein verteiltem Kupfer auf einem γ-Al₂O₃-Träger, mit dem Acetylen aus einem Butadien-haltigen Gemisch praktisch vollständig entfernt wird und der Butadienverlust im Bereich von höchstens 1% liegt.

1,3-Butadien dient z.B. als wichtiges Ausgangsprodukt für die Herstellung von Adipodinitril, aus dem z.B. α,ω-Alkylendiamine hergestellt werden können, wichtige Komponenten für die großtechnische Herstellung von Polyamiden (Nylon). Zur Herstellung dieser Diamine geht man im allgemeinen von entsprechenden Dinitrilen aus und unterwirft diese einer Hydrierung. So verlaufen alle industriell genutzten Verfahren zur Herstellung des 1,6-Diaminohexans über die Zwischenstufe des Adipinsäuredinitrils, von dem jährlich weltweit etwa 1.0 Mio. Tonnen produziert werden. In K. Weissermel, H.-J. Arpe, Industrielle Organische Chemie, 4. Auflage, VCH Weinheim, S. 266 ff. sind vier prinzipiell unterschiedliche Routen zur Herstellung von Adipinsäuredinitril beschrieben:
1. die dehydratisierende Aminierung der Adipinsäure mit Ammoniak in der Flüssig- oder Gasphase über intermediär gebildetes Diamid;
2. die indirekte Hydrocyanierung des 1,3-Butadiens über die Zwischenstufe der 1,4-Dichlorbutene;
3. die Hydrodimerisierung von Acrylnitril in einem elektrochemischen Prozess; und
4. die direkte Hydrocyanierung von 1,3-Butadien mit Cyanwasserstoff.

Nach dem letztgenannten Verfahren erhält man in einer ersten Stufe durch Monoaddition ein Gemisch isomerer Pentennitrile und Methylbutennitrile, das in einer zweiten Stufe zu vorwiegend 3-und 4-Pentennitril isomerisiert wird. Anschließend wird in einer dritten Stufe durch anti-Markownikow-Cyanwasserstoffaddition an 4-Pentennitril das Adipinsäuredinitril gebildet. Die Umsetzung erfolgt dabei in der Flüssigphase in einem Lösungsmittel, wie z. B. Tetrahydrofuran, bei einer Temperatur im Bereich von 30 - 150 °C und drucklos. Dabei werden als Katalysatoren Nickelkomplexe mit Phosphin- bzw. Phosphit-Liganden und Metallsalz-Promotoren verwendet. In dem oben genannten Review findet sich kein Hinweis auf eine mögliche Brauchbarkeit eines industriellen C₄-Schnittes anstelle von reinem 1,3-Butadien als Edukt.

In "Applied Homogeneous Catalysis with Organometalic Compounds", Bd. 1, VCH Weinheim, S. 465 ff. wird allgemein die heterogen und homogen katalysierte Addition von Cyanwasserstoff an Olefine beschrieben. Dabei werden vor allem Katalysatoren auf Basis von Phosphin- und Phosphit-Komplexen des Nickels und Palladiums verwendet, die eine hohe Produktselektivität, verbesserte Umsätze und verringerte Reaktionszeiten ermöglichen. Zur Herstellung von Adipinsäuredinitril durch Hydrocyanierung von Butadien werden vorwiegend Nickel(0)-Phosphitkatalysatoren, ggf. in Gegenwart einer Lewis-Säure als Promotor verwendet. Allgemein läßt sich die Reaktion in die drei Schritte gliedern: 1. Synthese von Mononitrilen durch Hydrocyanierung von 1,3-Butadien; 2. Isomerisierung; 3. Synthese von Dinitrilen. Bei der Bildung des Monoadditionsproduktes erhält man ein Isomerengemisch aus 3-Pentennitril und 2-Methyl-3-butennitril, wobei die Selektivität bezüglich des linearen 3-Pentennitrils in Abhängigkeit vom verwendeten zum Katalysator etwa 70 bis 90 % beträgt. Wird dieser erste Reaktionsschritt in Abwesenheit von Lewis-Säuren durchgeführt, so findet im allgemeinen keine Zweitaddition von Cyanwasserstoff statt und das erhaltene Produktgemisch kann einer Isomerisierung unter Verwendung der gleichen Katalysatorsysteme wie im ersten Reaktionsschritt, diesmal in Gegenwart einer Lewis-Säure, wie z. B. ZnCl₂, als Promotor unterzogen werden. Dabei findet zum einen Isomerisierung von 2-Methyl-3-butennitril zu 3-Pentennitril und zum anderen die Isomerisierung von 3-Pentennitril zu den verschiedenen n-Cyannitrilen statt. In dieser Publikation ist erwähnt, daß das thermodynamisch stabilste Isomer, das 2-Pentennitril, bei dem die C,N-Dreifachbindung mit der C,C-Doppelbindung in Konjugation steht, die Reaktion inhibiert, da es als Katalysatorgift wirkt. Die gewünschte Isomerisierung zu 4-Pentennitril wird nur dadurch ermöglicht, daß 3-Pentennitril wesentlich schneller zu 4-Pentennitril als zu 2-Pentennitril isomerisiert wird.

Die EP-A-0 274 401 beschreibt ein Verfahren zur Hydrocyanierung von reinem Butadien unter Verwendung eines Nickelkatalysators, der eine Mischung aus Phenyl- und m,p-Tolyl-Phosphitliganden aufweist.

Die EP-A-315 551 beschreibt ein Verfahren zur Hydrocyanierung reiner Diene, wie z.B. 1,3-Butadien, 1,3-Hexadien, etc., unter Katalyse mit einem Ni(0)-Katalysator, der eine Säure als Promotor enthält.

Die US-A-4,434,316 beschreibt ein Verfahren zur Abtrennung der Alkene aus einer Mischung von Alkenen und Alkandienen, wobei die Mischung mit Cyanwasserstoff in Gegenwart eines Nickel(0)-Romplexes als Katalysator umgesetzt wird. Dabei reagieren vorzugsweise die Alkadiene unter Bildung der entsprechenden Nitrile und können von den nicht umgesetzten Alkenen abgetrennt werden. Eine solche Alken-Alkadien-Trennung ist z. B. bei technischen Verfahren zur Herstellung von Dinitrilen erforderlich, um die Alkene, die keine Dinitrile bilden können, von den Alkadienen zu trennen. Das beschriebene Verfahren eignet sich zur Trennung von Alkenen mit 2 bis 5 Kohlenstoffatomen, wie z. B. Ethylen, Propylen, Butenen und Propenen von Alkadienen mit 3 bis 8 Kohlenstoffatomen, wie z.B. Propadien, Butadien, Pentadien, Hexadien und Octadien. Die Anwesenheit von acetylenisch und ethylenisch-acetylenisch ungesättigten Kohlenwasserstoffen wird für das beschriebene Trennungsverfahren nicht als nachteilig angesehen. Auf die Möglichkeit zur Hydrocyanierung eines 1,3-Butadien-haltigen Kohlenwasserstoffgemisches und speziell eines C₄₋Schnittes aus der Erdölraffination zur Herstellung von C₅-Monoolefingemischen mit Nitrilfunktion findet sich in der Schrift kein Hinweis.

Die aus dem Stand der Technik bekannten Verfahren zur Hydrocyanierung von 1,3-Butadien-haltigen Kohlenwasserstoffgemischen besitzen jedoch den Nachteil, daß als unerwünschte Nebenprodukte konjugierte Buten- und/oder Pentennitrile anfallen. Diese sind destillativ nur unzureichend von den nicht-konjugierten Wertprodukten 3-Pentennitril und 2-Methyl-3-butennitril abtrennbar, nicht weiter zu Adipodinitril hydrocyanierbar und stellen außerdem ausgesprochene Katalysatorgifte dar.

Der vorliegenden Erfindung liegt die Aufgabe zugrunde, ein Verfahren zur Herstellung von Gemischen monoolefinischer C₅-Mononitrile bereitzustellen, das die oben geschilderten Nachteile nicht aufweist und eine wirtschaftliche Produktion von Adipodinitril ermöglicht.

Überraschenderweise wurde nun gefunden, daß die Aufgabe durch ein Verfahren der eingangs bezeichneten Art gelöst wird, wobei man zur Hydrocyanierung ein 1,3-Butadien-haltiges Kohlenwasserstoffgemisch verwendet, welches im wesentlichen frei von störenden Komponenten, wie Alkinen und 1,2-Dienen ist. Es wurde nämlich überraschenderweise festgestellt, daß die oben beschriebenen Nachteile vermieden werden können, wenn man insbesondere den Anteil von Alkinen und 1,2-Dienen, die bei der Hydrocyanierung die unerwünschten konjugierten Nitrile bilden, in dem zur Hydrocyanierung eingesetzten Kohlenwasserstoffgemisch verringert. Die dabei erfindungsgemäß hergestellten neuen Nitrilgemische eignen sich deshalb z. B. nach weiterer Aufarbeitung und Isomerisierung besonders als Zwischenprodukte für die Herstellung von Adipinsäuredinitril durch Addition eines weiteren Äquivalents Cyanwasserstoff.

Ein erster Gegenstand der Erfindung betrifft somit ein Verfahren zur Herstellung von Gemischen monoolefinischer C₅-Mononitrile mit nichtkonjugierter C=C- und C ≡ N-Bindung durch katalytische Hydrocyanierung eines 1,3-Butadien-haltigen Kohlenwasserstoffgemisches, das dadurch gekennzeichnet ist, daß man das Kohlenwasserstoffgemisch mit Wasserstoff in Gegenwart eines Hydrierungskatalysators umsetzt, der befähigt ist, Alkine und 1,2-Diene mit hoher Selektivität zu hydrieren, ohne den Gehalt an 1,3-Butadien wesentlich zu verringern, um aus dem Kohlenwasserstoffgemisch Alkine, 1,2-Diene und Gemische davon teilweise oder vollständig zu entfernen, so daß der Anteil derjenigen Komponenten, welche die katalytische Hydrocyanierung beeinträchtigen, verringert wird, und man das so erhaltene Gemisch anschließend katalytisch hydrocyaniert.

Besonders bei der Hydrocyanierung eines C₄-Schnittes, welcher nicht im wesentlichen frei von Alkinen, wie z. B. Propin oder Butin, von 1,2-Dienen, wie z. B. Propadien, und von Alkeninen, wie z. B. Vinylacetylen, ist, werden Produkte erhalten, bei denen eine C=C-Doppelbindung in Konjugation mit der C≡N-Bindung steht. Wie bereits zuvor erwähnt, ist aus "Applied Homogeneous Catalysis with Organometalic Compounds", Bd. 1, VCH Weinheim, S. 479 bekannt, daß das bei der Isomerisierung von 2-Methyl-3-butennitril und 3-Pentennitril entstehende, konjugierte 2-Pentennitril als ein Reaktionsinhibitor für die Zweitaddition von Cyanwasserstoff zu Adipinsäuredinitril wirkt. Es wurde festgestellt, daß die oben genannten, bei der Hydrocyanierung eines nicht vorbehandelten C₄-Schnittes erhaltenen konjugierten Nitrile auch als Katalysatorgifte für den ersten Reaktionsschritt der Adipinsäureherstellung, die Monoaddition von Cyanwasserstoff, wirken. Unmittelbare Folge davon sind eine deutlich herabgesetzte katalytische Aktivität des homogen vorliegenden Nickelkatalysators, verringerte Umsätze und verringerte Nickelwiederfindungsraten bei der Katalysatorrückgewinnung. Diese Nachteile können, wie im experimentellen Teil gezeigt, vermieden werden.

Insbesondere ist erfindungsgemäß zu beobachten, daß nach selektiver Hydrierung der Alkin- und 1,2-Dienanteile deutlich höhere HCN-Umsätze bei der Hydrocyanierung erzielt werden, die Nickel-Bilanz deutlich verbessert ist und selbst bei geringerem 1,3-Butadien-Überschuß bessere 1,3-Butadien-Umsätze erzielt werden können.

Vorzugsweise wird zur Herstellung von monoolefinischen C₅-Mononitrilen nach dem erfindungsgemäßen Verfahren ein großtechnisch anfallendes Kohlenwasserstoffgemisch verwendet, das einen hohen Anteil an 1,3-Butadien aufweist. Dazu zählt z.B. der bei der Erdölverarbeitung durch Steamcracken von Naphtha anfallende C₄-Schnitt, der produktionsbedingt auch immer Alkine und 1,2-Diene enthält. Zur Entfernung dieser Komponenten wird der C₄-Schnitt vor der Addition von Cyanwasserstoff einer katalytischen Teilhydrierung unterzogen. Diese Teilhydrierung erfolgt erfindungsgemäß in Gegenwart eines Hydrierungskatalysators, der befähigt ist, Alkine und 1,2-Diene selektiv neben anderen Dienen und Monoolefinen zu hydrieren.

Besonders geeignet für die Verwendung im vorliegenden Verfahren ist z. B. ein an 1,3-Butadien angereichertes Gemisch (C₄-Schnitt), welches einen 1,3-Butadiengehalt von mindestens 10 Vol.-%, bevorzugt mindestens 25 Vol.-%, insbesondere mindestens 43 Vol.-% aufweist. Vorzugsweise werden dabei Gemisch mit einem möglichst hohen Butadiengehalt von mindestens 35 Vol.-%, bevorzugt mindestens 40 Vol.-%, insbesondere mindestens 42 Vol.-% verwendet.

Nach einer speziellen Ausführungsform des erfindungsgemäßen Verfahrens wird ein großtechnisch anfallender C₄-Schnitt verwendet, der die folgenden Kohlenwasserstoffe enthält:
10 bis 50 Vol.-%, bevorzugt 25 bis 47 Vol.-% 1,3-Butadien;
10 bis 35 Vol.-%, bevorzugt 15 bis 30 Vol.-% Isobuten;
2 bis 30 Vol.-%, bevorzugt 5 bis 20 Vol.-% 1-Buten;
1 bis 20 Vol.-%, bevorzugt 3 bis 15 Vol.-% n-Butan
1 bis 15 Vol.-%, bevorzugt 2 bis 10 Vol.-% trans-2-Buten;
1 bis 15 Vol.-%, bevorzugt 2 bis 10 Vol.-% Isobutan;
1 bis 15 Vol.-%, bevorzugt 2 bis 10 Vol.-% cis-2-Buten
sowie zusammen etwa 0,1 bis 5,0 Vol.-%, bevorzugt 0,2 bis 2,5 Vol.-% Alkine und 1,2-Diene, wie z. B. Vinylacetylen, 1-Butin, Propin, Propadien (Allen) etc., und Spurengase, wie z. B. Propan, Cyclopropan, Propen, iso-Pentan, n-Pentan etc., im Bereich von jeweils etwa 1 bis 500 ppm.

Nach einer weiteren speziellen Ausführungsform des erfindungsgemäßen Verfahrens wird ein großtechnisch anfallender C₄-Schnitt verwendet, der die folgenden Kohlenwasserstoffe enthält:
35 bis 50 Vol.-%, bevorzugt 43 bis 47 Vol.-% 1,3-Butadien;
17 bis 35 Vol.-%, bevorzugt 20 bis 30 Vol.-% Isobuten;
8 bis 18 Vol.-%, bevorzugt 11 bis 15 Vol.-% 1-Buten;
3 bis 13 Vol.-%, bevorzugt 6 bis 10 Vol.-% n-Butan
1 bis 7 Vol.-%, bevorzugt 3 bis 5 Vol.-% trans-2-Buten;
1 bis 5 Vol.-%, bevorzugt 2 bis 4 Vol.-% Isobutan;
1 bis 5 Vol.-%, bevorzugt 2 bis 5 Vol.-% cis-2-Buten
sowie zusammen etwa 0,5 bis 1,4 Vol.-% Alkine und 1,2-Diene, wie z. B. Vinylacetylen, 1-Butin, Propin, Propadien (Allen) etc., und Spurengase, wie z. B. Propan, Cyclopropan, Propen, iso-Pentan, n-Pentan etc., im Bereich von jeweils etwa 5 bis 250 ppm.

Vorzugsweise wird der C₄-Schnitt einer selektiven Hydrierung unterzogen, die im wesentlichen nur die enthaltenen Alkine und 1,2-Diene erfaßt. So beträgt der Gesamtanteil an diesen Alkinen und 1,2-Dienen im Kohlenwasserstoff nach der selektiven Hydrierung höchstens noch etwa 1000 ppm, bevorzugt höchstens etwa 800 ppm, insbesondere höchstens etwa 600 ppm. Nach einer speziellen Ausführungsform beträgt der Gesamtanteil an disen Komponenten nach der Hydrierung höchstens noch 100 ppm. Des weiteren wird vor allem der Anteil an 1,3-Butadien nur wenig verringert, im allgemeinen um höchstens etwa 10 Vol.-%, bevorzugt um höchstens 5 Vol.-%, insbesondere um höchstens 1 Vol.-%.

Nach einer speziellen Ausführungsform des erfindungsgemäßen Verfahrens wird nach der selektiven Hydrierung ein Kohlenwasserstoffgemisch erhalten, das die folgenden Kohlenwasserstoffe enthält:
10 bis 50 Vol.-%, bevorzugt 25 bis 47 Vol.-% 1,3-Butadien;
10 bis 35 Vol.-%, bevorzugt 15 bis 30 Vol.-% Isobuten;
2 bis 30 Vol.-%, bevorzugt 5 bis 25 Vol.-% 1-Buten;
1 bis 15 Vol.-%, bevorzugt 2 bis 10 Vol.-% trans-2-Buten;
1 bis 20 Vol.-%, bevorzugt 3 bis 15 Vol.-% n-Butan;
1 bis 15 Vol.-%, bevorzugt 2 bis 10 Vol.-% cis-2-Buten;
1 bis 15 Vol.-%, bevorzugt 2 bis 10 Vol.-% Isobutan;
sowie
10 bis 500 ppm 1-Butin;
10 bis 1000 ppm Vinylacetylen;
10 bis 250 ppm Propadien;
5 bis 250 ppm Propin;
und die oben erwähnten Spurengase im Bereich von jeweils 5 bis 500 ppm.

Nach einer weiteren speziellen Ausführungsform des erfindungsgemäßen Verfahrens wird nach der selektiven Hydrierung ein Kohlenwasserstoffgemisch erhalten, das die folgenden Kohlenwasserstoffe enthält:
30 bis 47 Vol.-%, bevorzugt 35 bis 44 Vol.-% 1,3-Butadien;
15 bis 35 Vol.-%, bevorzugt 21 bis 30 Vol.-% Isobuten;
15 bis 25 Vol.-%, bevorzugt 18 bis 22 Vol.-% 1-Buten;
4 bis 10 Vol.-% bevorzugt 6 bis 8 Vol.-% trans-2-Buten;
1,5 bis 7,5 Vol.-%, bevorzugt 3,5 bis 5,5 Vol.-% n-Butan;
1,5 bis 7,5 Vol.-%, bevorzugt 3,3 bis 5,3 Vol.-% cis-2-Buten;
0,4 bis 1,4 Vol.-%, bevorzugt 0,7 bis 1,2 Vol.-% Isobutan;
sowie
100 bis 250 ppm 1-Butin;
80 bis 250 ppm Vinylacetylen;
30 bis 60 ppm Propadien;
10 bis 50 ppm Propin;
und die oben erwähnten Spurengase im Bereich von jeweils 5 bis 500 ppm.

Geeignete Katalysatoren für die selektive Hydrierung sind aus dem Stand der Technik bekannt und umfassen übliche homogene und heterogene Hydrierkatalysatorsysteme. Vorzugsweise basieren die für das erfindungsgemäße Verfahren geeigneten Katalysatoren auf einem Übergangsmetall der 8. oder 1. Nebengruppe, wobei vorzugsweise Katalysatoren auf Basis von Ni, Pd, Pt, Ru oder Cu verwendet werden. Insbesondere bevorzugt werden Katalysatoren auf Basis von Cu oder Pd verwendet.

Geeignete heterogene Katalysatorsysteme umfassen im allgemeinen eine der zuvor genannten Übergangsmetallverbindungen auf einem inerten Träger. Geeignete anorganische Träger sind die hierfür üblichen Oxide, insbesondere Silicium- und Aluminiumoxide, Alumosilikate, Zeolithe, Carbide, Nitride etc. und deren Mischungen. Bevorzugt werden als Träger Al₂O₃, SiO₂ und deren Mischungen verwendet. Insbesondere handelt es sich bei den im erfindungsgemäßen Verfahren verwendeten heterogenen Katalysatoren um die in den US-A-4,587,369; US-A-4,704,492 und US-A-4,493,906 beschriebenen, auf die hier in vollem Umfang Bezug genommen wird. Weiterhin geeignete Katalysatorsysteme auf Cu-Basis werden von der Fa. Dow Chemical als KLP-Katalysator vertrieben.

Die Addition von Cyanwasserstoff an den vorbehandelten, teilhydrierten C₄-Schnitt, und speziell an das darin enthaltene 1,3-Butadien kann kontinuierlich, semikontinuierlich oder diskontinuierlich erfolgen.

Die kontinuierliche Cyanwasserstoffaddition umfaßt formal:
a) Einspeisung von teilhydriertem C₄-Schnitt, Cyanwasserstoff und Hydrocyanierungskatalysator in einen Reaktor,
b) Umsetzung des Gemisches bei erhöhter Temperatur und erhöhtem Druck,
c) Entfernung von nicht umgesetztem Cyanwasserstoff und 1,3-Butadien aus dem umgesetzten Gemisch,
d) Rückführung des gasförmigen Reaktoraustrags, ggf nach destillativer Aufarbeitung in den Reaktor,
e) destillative Aufarbeitung des flüssigen und festen Reaktoraustrags und Rückführung des dabei zurückgewonnenen Katalysators in den Reaktor.

Geeignete Reaktoren für die kontinuierliche Umsetzung sind dem Fachmann bekannt und werden z. B. in Ullmanns Encyklopädie der technischen Chemie, Bd. 1, 3. Aufl., 1951, S. 743 ff. beschrieben. Vorzugsweise wird für die kontinuierliche Variante des erfindungsgemäßen Verfahrens eine Rührkesselkassade, bevorzugt aus zwei Stufen, oder ein Rohrreaktor verwendet. Für die Schritte a) bis e) gilt vorzugsweise folgendes zu beachten:

### Schritt a):

Die Einspeisung der drei Komponenten des Reaktionsgemisches, Cyanwasserstoff, teilhydrierter C₄-Schnitt und Katalysator erfolgt im allgemeinen in separaten Zuläufen nach Maßgaben des jeweiligen Verbrauchs. Wird anstelle eines homogenen Katalysators, der z. B. separat als Lösung in einem geeigneten Lösungsmittel oder gemeinsam mit einem der übrigen Zuläufe in den Reaktor geführt werden kann, ein heterogener Katalysator verwendet, so kann dieser auch in geeigneter Form im Reaktor vorgelegt werden.

### Schritt b):

Die Umsetzung des teilhydrierten C₄-Schnittes und speziell die Monoaddition von Cyanwasserstoff an das enthaltene 1,3-Butadien erfolgt vorzugsweise bei erhöhter Temperatur und bei erhöhtem Druck. Dabei liegt die Temperatur im allgemeinen in einem Bereich von etwa 20 bis etwa 200 °C, bevorzugt etwa 70 bis etwa 120 °C. Der Reaktionsdruck entspricht im allgemeinen dem von der Reaktionsmischung unter den Reaktionstemperaturen aufgebauten Druck und liegt in einem Bereich von etwa 1 bis 200 bar, bevorzugt etwa 2 bis 100 bar, insbesondere etwa 5 bis 20 bar.

### Schritt c):

Im Anschluß an die Reaktion werden nichtumgesetztes 1,3-Butadien und Cyanwasserstoff sowie die weiteren im Reaktionsgemisch enthaltenen gasförmigen Komponenten aus dem Reaktionsgemisch entfernt, destillativ getrennt und/oder zurückgeführt (Kreisstrom). Dies kann in bekannter Weise z.B. durch Strippen in einer dafür üblichen Kolonne erfolgen.

### Schritt d):

In Abhängigkeit von der Konzentration des noch enthaltenen, nichtumgesetzten 1,3-Butadiens kann der gasförmige Austrag, gegebenenfalls nach vorheriger destillativer Aufarbeitung, wieder in den Reaktor zurückgeführt werden. Zur Entfernung der restlichen Blausäure kann das Abgas mit wässriger Lauge gewaschen werden. Geeignete Waschlaugen sind die Alkalihydroxide, wie z. B. KOH und NaOH. Das im Abgas enthaltene 1,3-Butadien-freie C₄-Gemisch (Raffinat 1) kann der weiteren petrochemischen Aufarbeitung zugeführt werden.

### Schritt e):

Der flüssige und/oder feste Reaktoraustrag wird zur Gewinnung der Wertprodukte 3-Pentennitril und 2-Methyl-3-butennitril sowie zur Rückgewinnung des enthaltenen Katalysators einer destillativen Aufarbeitung unterzogen. Der dabei zurückgewonnene aktive Katalysator wird in den Reaktor zurückgeführt.

Nach einer speziellen Ausführungsform der kontinuierlichen Verfahrensvariante wird eine zweistufige Rührkesselkaskade verwendet, wobei die Verweilzeit etwa 10 bis 120 Minuten pro Stufe, bevorzugt etwa 20 bis 60 Minuten pro Stufe beträgt.

Gemäß einer weiteren geeigneten Variante des erfindungsgemäßen Verfahrens erfolgt die Addition des Cyanwasserstoffs an den teilhydrierten C₄-Schnitt semikontinuierlich.

Das semikontinuierliche Verfahren umfaßt:
a) Befüllen eines druckfesten Reaktors mit teilhydriertem C₄-Schnitt, Cyanwasserstoff und Hydrocyanierungskatalysator sowie ggf. einem Lösungsmittel,
b) Umsetzung des Gemisches bei erhöhter Temperatur und erhöhtem Druck, wobei bei semikontinuierlicher Fahrweise Cyanwasserstoff nach Maßgabe seines Verbrauchs eingespeist wird,
c) Vervollständigung des Umsatzes durch Nachreagieren und anschließende Aufarbeitung.

Geeignete druckfeste Reaktoren sind dem Fachmann bekannt und werden z. B. in Ullmanns Encyklopädie der technischen Chemie, Bd. 1, 3. Auflage, 1951, S. 769 ff. beschrieben. Im allgemeinen wird für das erfindungsgemäße Verfahren ein Autoklav verwendet, der gewünschtenfalls mit einer Rührvorrichtung und einer Innenauskleidung, z. B. aus Glas, versehen sein kann. Für die obigen Schritte gilt vorzugsweise folgendes zu beachten:

### Schritt a):

Der druckfeste Reaktor wird vor Beginn der Reaktion mit dem teilhydrierten C₄-Schnitt, Cyanwasserstoff einem Hydrocyanierungskatalysator sowie ggf. einem Lösungmittel befüllt. Geeignete Lösungsmittel sind z. B. aromatische Kohlenwasserstoffe, wie Toluol und Xylol, oder Tetrahydrofuran.

### Schritt b):

Die Umsetzung des Gemisches erfolgt im allgemeinen bei erhöhter Temperatur und erhöhtem Druck. Dabei liegt die Reaktionstemperatur im allgemeinen in einem Bereich von etwa 20 bis 200°C, bevorzugt etwa 70 bis 120°C Der Druck liegt im allgemeinen in einem Bereich von etwa 1 bis 200 bar, bevorzugt etwa 5 bis 20 bar. Dabei wird während der Reaktion Cyanwasserstoff nach Maßgabe seines Verbrauchs eingespeist, wobei der Druck im Autoklaven im wesentlichen konstant bleibt. Die Reaktionszeit beträgt etwa 30 Minuten bis 5 Stunden, bevorzugt etwa 1 Stunde bis 3 Stunden.

### Schritt c):

Zur Vervollständigung des Umsatzes kann sich an die Reaktionszeit eine Nachreaktionszeit von 0 Minuten bis etwa 5 Stunden, bevorzugt etwa 1 Stunde bis 3 Stunden anschließen, in der kein Cyanwasserstoff mehr in den Autoklaven eingespeist wird. Die Temperatur wird in dieser Zeit im wesentlichen konstant auf der zuvor eingestellten Reaktionstemperatur belassen. Die Aufarbeitung erfolgt nach gängigen Verfahren und umfaßt die Abtrennung des nicht umgesetzten 1,3-Butadiens und des nicht umgesetzten Cyanwasserstoffs, z. B. durch Waschen oder Extrahieren und die destillative Aufarbeitung des übrigen Reaktionsgemisches zur Abtrennung der Wertprodukte und Rückgewinnung des noch aktiven Katalysators.

Gemäß einer weiteren geeigneten Variante des erfindungsgemäßen Verfahrens erfolgt die Addition des Cyanwasserstoffs an den teilhydrierten C₄-Schnitt diskontinuierlich. Dabei werden im wesentlichen die bei semikontinuierlichen Verfahren beschriebenen Reaktionsbedingungen eingehalten, wobei in Schritt b) kein zusätzlicher Cyanwasserstoff eingespeist wird.

Geeignete Katalysatoren für die Hydrocyanierung sind aus dem Stand der Technik bekannt und umfassen heterogene und vorzugsweise homogene Katalysatorsysteme. Im allgemeinen basieren die für das erfindungsgemäße Verfahren geeigneten Katalysatoren auf einem Übergangsmetall der 8. Nebengruppe und umfassen ggf. ein Metallsalz als Promotor. Vorzugsweise wird für die Monoaddition von Cyanwasserstoff ein Katalysatorsystem ohne Zusatz eines Promotors verwendet.

Bevorzugt wird für das erfindungsgemäße Verfahren mindestens ein homogener Katalysator, ausgewählt unter Salzen oder Komplexverbindungen des Nickels, verwendet. Insbesondere eignen sich Ni(0)-Komplexe mit Phosphin-, Phosphinit-, Phosphonit- oder vorzugsweise Phosphit-Liganden.
Geignet sind Ni-, Phosphin-, Phosphinit-, Phosphonit- bzw. Phosphit-Komplexe der allgemeinen Formel NiL₄, worin L für PR₃, P(OR)R₂, P(OR)₂R oder P(OR)₃ steht und R für einen Alkyl-, Cycloalkyl- oder Arylrest, bevorzugt für Phenyl oder m,p-Tolyl, steht. Dabei können auch Gemische unterschiedlicher Reste R verwendet werden. Nach einer bevorzugten Ausführungsform erfolgt die Umsetzung in Gegenwart eines Ni(0)-Komplexes, welcher mindestens einen zwei- oder mehrzähnigen Phosphin-, Phosphinit-, Phosphonit- oder Phosphitliganden umfaßt. Geeignete Phosphit-Chelatbildner sind in der WO 96/22968, US-A-5,484,902, WO 96/11182, US-A-5,523,453, WO 95/30680, WO 95/28228 und WO 95/14659 beschrieben, auf die hier in vollem Umfang Bezug genommen wird.

Die vorgenannten Katalysatoren können gewünschtenfalls in Kombination mit einem Promotor verwendet werden. Vorzugsweise wird als Promotor eine Lewis-Säure, wie z.B. AlCl₃ oder ZnCl₂ verwendet. Bevorzugt werden die Katalysatoren für die Monoaddition zu Cyanwasserstoff ohne Zusatz eines Promotors verwendet.

Gemäß einer speziellen Ausführungsform des erfindungsgemäßen Verfahrens kann einer der zuvor genannten Liganden im Überschuss als Lösungsmittel verwendet werden. Gegebenenfalls kann auch das Produkt 3-Pentennitril als Lösungsmittel fungieren.

Allgemein läßt sich die Herstellung von Adipinsäuredinitril aus einem Butadien-haltigen Gemisch durch Addition von 2 Moläquivalenten Cyanwasserstoff in drei Schritte gliedern:
1. Herstellung von C₅-Monoolefingemischen mit Nitrilfunktion.
2. Isomerisierung des in diesen Gemischen enthaltenen 2-Methyl-3-butennitrils zu 3-Pentennitril und Isomerisierung des so gebildeten und des in den Gemischen bereits aus Schritt 1 enthaltenen 3-Pentennitrils zu verschiedenen n-Pentennitrilen. Dabei soll ein möglichst hoher Anteil an 3-Pentennitril und ein möglichst geringer Anteil an konjugiertem und als Katalysatorgift wirksamen 2-Pentennitril und 2-Methyl-2-butennitril gebildet werden.
3. Herstellung von Adipinsäuredinitril durch Addition von Cyanwasserstoff an das in Schritt 2 gebildete 3-Pentennitril welches zuvor "in situ" zu 4-Pentennitril isomerisiert wird. Als Nebenprodukte treten dabei z. B. 2-Methyl-glutarodinitril aus der Markownikow-Addition von Cyanwasserstoff an 4-Pentennitril oder der anti-Markownikow-Addition von Cyanwasserstoff an 3-Pentennitril und Ethylsuccinodinitril aus der Markownikow-Addition von Cyanwasserstoff an 3-Pentennitril auf.

Vorteilhafterweise eignen sich die für die erfindungsgemäße Herstellung von monoolefinischen Nitrilen in Schritt 1 bevorzugt verwendeten Nickelphosphit-Katalysatoren auch für die Stellungsund Doppelbindungsisomerisierung in Schritt 2 und die Zweitaddition von Cyanwasserstoff in Schritt 3. Nach einer geeigneten Vorgehensweise erfolgt dabei z.B. die Monoaddition nach Schritt 1 in Abwesenheit von Promotoren, z.B. Lewis-Säuren wie ZnCl₂, die Isomerisierung nach Schritt 2 in Anwesenheit von Lewis-Säuren, wobei die Ausbeute im Hinblick auf 3-Pentennitril optimiert wird und die Zweitaddition in Schritt 3 ggf. unter Ersetzung des ZnCl₂ durch Triphenylbor, um die Selektivität der Reaktion bzgl. Adipinsäuredinitril zu optimieren.

Das erfindungsgemäße Verfahren zur Herstellung monoolefinischer C₅-Nitrile aus einem im wesentlichen Alkin- und 1,2-Dien-freien Kohlenwasserstoffgemisch ermöglicht höhere Cyanwasserstoffumsätze und höhere 1,3-Butadien-Umsätze, als sie bei der Hydrocyanierung eines nichtvorbehandelten C₄-Schnittes erzielt werden. Dabei liegen die erreichten Reaktionsumsätze bezogen auf Cyanwasserstoff bei mindestens 90 %, bevorzugt bei mindestens 95 %, insbesondere bevorzugt bei mindestens 98 % und speziell bei mindestens 99 %. Bei semikontinuierlicher Reaktionsführung werden unter ansonsten identischen Reaktionsbedingungen mit nichtvorbehandeltem C₄-Schnitt deutlich niedrigere Reaktionsumsätze bezogen auf Cyanwasserstoff erzielt.

Bei der für großtechnische Prozesse bevorzugten kontinuierlichen Reaktionsführung werden z.B. unter ansonsten zu dem erfindungsgemäßen Verfahren identischen Reaktionsbedingungen mit nichtvorbehandeltem C₄-Schnitt Reaktionsumsätze bezogen auf Cyanwasserstoff im Bereich von etwa 98 % erzielt, gegenüber im wesentlichen vollständigen Umsätzen im Bereich von z. B. 99,8 % bei der Verwendung von erfindungsgemäß vorbehandeltem C₄-Schnitt. Da das kontinuierliche Verfahren unter Zurückführung des aktiven Nickelkatalysators gefahren wird, und der Katalysator dem Reaktionsgemisch nur in geringen Mengen zugeführt wird, hat bereits ein geringfügiger Cyanwasserstoffüberschuß, der aus einem unvollständigen Umsatz resultiert, einen stark negativen Einfluss auf die Nickelbilanz der Reaktion. So weist das erfindungsgemäße kontinuierliche Verfahren überraschenderweise eine höhere Katalysatorrückführungsrate und somit längere Katalysatorstandzeiten auf als bekannte Verfahren. Dies ist auch unter ökologischen Aspekten relevant, da das aus dem aktiven Katalysator mit Cyanwasserstoff gebildete Nickelcyanid stark giftig ist und unter hohen Kosten aufgearbeitet oder entsorgt werden muß.

Wird nach dem erfindungsgemäßen Verfahren ein wie zuvor beschriebener teilhydrierter C₄-Schnitt einer Hydrocyanierung unterzogen, so wird im wesentlichen nur das 1,3-Butadien monohydrocyaniert. Nach Gasraumanalysen werden die im vorbehandelten C₄-Schnitt enthaltenen Monoolefine nicht mit Cyanwasserstoff umgesetzt, sondern z.T. isomerisiert. So kann z.B. eine Isomerisierung des enthaltenen 1-Butens zu cis- und trans-2-Buten beobachtet werden. Das erhaltene Produktgemisch umfaßt im allgemeinen isomere Pentennitrile und Methylbutennitrile, wie 3-Pentennitril, 2-Pentennitril, 4-Pentennitril, 2-Methyl-2-butennitril, 2-Methyl-3-butennitril, etc.

Vorzugsweise wird ein hoher Anteil an 4-Pentennitril sowie an zu 4-Pentennitril isomerisierbaren Produkten, wie 3-Pentennitril und 2-Methyl-3-butennitril erhalten. Desweiteren ist der Anteil an kojugiertem 2-Pentennitril, welches als Katalysatorgift wirkt gering und liegt vorzugsweise unterhalb von 1 Gew.-%, insbesondere bei etwa 0,1 bis 0,2 Gew.-%, bezogen auf eingesetzten C₄-Schnitt.

Das Produktmengenverhältnis von 3-Pentennitril zu 2-Methyl-3-butennitril beträgt im allgemeinen etwa 1,5:1 bis 2,5:1, bevorzugt 1,8:1 bis 2,3:1.

Wie Gasraumanalysen belegen, werden bei der Verwendung von nicht teilhydriertem C₄-Schnitt für die Hydrocyanierung nicht nur das 1,3-Butadien, sondern auch die enthaltenen Alkine und 1,2-Diene hydrocyaniert, wobei Produkte mit einer C,C-Doppelbindung in Konjugation zur Nitrilfunktion gebildet werden. Aufgrund der Wirkung dieser Verbindungen als Katalysatorgifte sind die Nickelwiederfindungsraten und die Katalysatorstandzeiten niedriger als im erfindungsgemäßen Verfahren. Geringere Katalysatoraktivitäten führen dabei zu niedrigeren Umsätzen.

Ein weiterer Gegenstand der Erfindung ist ein Verfahren zur Herstellung von Adipodinitril, das dadurch gekennzeichnet ist, daß man ein wie zuvor beschrieben hergestelltes Gemisch von C₅-Mononitrilen, gegebenenfalls nach weiterer Aufarbeitung oder Isomerisierung katalytisch hydrocyaniert.

Die Erfindung wird anhand der folgenden nicht einschränkenden Beispiele erläutert.

### Beispiele

Für die folgenden erfindungsgemäßen Beispiele wurde ein teilhydriertes Kohlenwasserstoffgemisch verwendet, welches enthält:
38,9 Vol.-% 1,3-Butadien,
22,4 Vol.-% Isobuten,
19,8 Vol.-% 1-Buten,
4,5 Vol.-% n-Butan,
7,05 Vol.-% trans-2-Buten,
0,89 Vol.-% Isobutan,
4,30 Vol.-% cis-2-Buten,
159 ppm Vinylacetylen,
214 ppm 1-Butin,
29 ppm Propin,
44 ppm Propadien,
156 ppm i-Pentan,
45 ppm Cyclopropan
333 ppm Propen,
6 ppm n-Pentan,
6 ppm Propan

Für die folgenden Vergleichsbeispiele wurde ein nicht hydriertes Kohlenwasserstoffgemisch verwendet, welches enthält:
45,2 Vol.-% 1,3-Butadien,
22,0 Vol.-% Isobuten,
12,7 Vol.-% 1-Buten,
8,2 Vol.-% n-Butan,
3,8 Vol.-% trans-2-Buten,
2,92 Vol.-% Isobutan,
2,64 Vol.-% cis-2-Buten,
0,621 Vol.-% Vinylacetylen,
0,128 Vol.-% 1-Butin,
671 ppm Propin,
357 ppm Propadien,
258 ppm i-Pentan,
86 ppm Cyclopropan
23 ppm Propen,
15 ppm n-Pentan,
6 ppm Propan

### Beispiel 1:

### kontinuierliche Hydrocyanierung von teilhydriertem C₄-Schnitt

### Katalysatorzusammensetzung:

25 Gew.-% Tetrakis(tri-m/p-tolylphosphit)Ni(0)
60 Gew.-% Tri-m/p-tolylphosphit
15 Gew.-% 3-Pentennitril/2-Methyl-3-butennitril

Teilhydrierter C₄-Schnitt, Katalysatorlösung und Cyanwasserstoff werden in eine zweistufige Rührkesselkaskade eingespeist (Druck: 15 bar, Temperatur Reaktor 1: 102°C, Temperatur Reaktor 2: 95°C, Verweilzeit: 40 min/Reaktor). Der Austrag wird Blausäure- und Butadien-frei gestrippt, der Gasraum analysiert und der gasförmige Austrag über einen NaOH-Waschturm gefahren. Flüssiger/fester Austrag werden analysiert, destillativ zur Abtrennung der Wertprodukte und Rückgewinnung des noch aktiven Katalysators aufgearbeitet und die so erhaltenen Produkte erneut analysiert. Die auf HCN bezogenen Umsätze werden durch Maßanalyse der Reaktorlösungen aus beiden Reaktoren bestimmt. Die Nickelwiederfindungsrate (Rückgewinnung von aktivem Katalysator) wird mittels Elementaranalyse bestimmt.

Nach Gasraumanalyse wird selektiv 1,3-Butadien hydrocyaniert; 1-Buten wird zu cis- und trans-2-Buten isomerisiert. Der relative prozentuale Anteil von i-Buten, i-Butan und n-Butan bleibt vor und nach der Umsetzung im Rahmen der Meßgenauigkeit gleich. Die Ergebnisse sind in Tabelle 1 zusammemgefaßt.

### Vergleichsbeispiel 1

### kontinuierliche Hydrocyanierung von nicht teilhydriertem C₄-Schnitt

Ein nicht teilhydriertes Kohlenwasserstoffgemisch der oben angegebenen Zusammensetzung wird analog zu Beispiel 1 umgesetzt. Die Ergebnisse sind ebenfalls in Tabelle 1 wiedergegeben.

### Vergleichsbeispiel 2

### kontinuierliche Hydrocyanierung von nicht teilhydriertem C₄-Schnitt

Ein nicht teilhydriertes Kohlenwasserstoffgemisch der oben angegebenen Zusammensetzung wird analog zu Beispiel 1 umgesetzt, wobei die Katalysatoreinspeisung von 2,72 mmol/h auf 4,85 mmol/h erhöht wird. Die Ergebnisse sind ebenfalls in Tabelle 1 wiedergegeben.

**Tabelle 1**

| kontinuierliche Hydrocyanierung von teilhydriertem und nicht teilhydriertem C₄-Schnitt | | | | |
|---|---|---|---|---|
| | | Beispiel 1 | Vergleichsbsp. 1 | Vergleichsbsp. 2 |
| feeds | Ni-Kat. [mmol/h] | 2,76 | 2,72 | 4,85 |
| | 1,3-Butadien (als C₄-Schnitt) [mmol/h] | 483 | 533 | 513 |
| | HCN [mmol/h] | 414 | 421 | 455 |
| Gewichtsverhältnisse [Gew.-%] | Butadien:HCN | 1,17:1 | 1,27:1 | 1,13:1 |
| | Ni:Ligand | 1:14 | 1:14 | 1:14 |
| | Turnover-Zahl | 151 | 154 | 94 |
| | Selektivität bzgl. Butadien [%] | 98 | 97 | 97 |
| | Produktverhältnis 3-PN:2-M-3BN¹⁾ [Gew.-%] | 2,0:1 | 1,95:1 | 2,1:1 |
| zurückgewonnene akt. Kat.-Menge Ni [Gew.-%] | | 89,0 | 66,0 | 80,5 |
| | | 78,0 | 61,0 | 72,1 |
| | | 90,0 | 61,3 | 65,3 |
| Umsatz | HCN [Gew.-%] | >99,8 | 98,0 | 99,0 |

### 1) 3-PN = 3-Pentennitril, 2-M-3-BN = 2-Methyl-3-butennitril

Wie das erfindungsgemäße Beispiel 1 und die Vergleichsbeispiele 1 und 2 eindeutig belegen ist der Cyanwasserstoffumsatz bei der Verwendung eines selektiv hydrierten Kohlenwasserstoffgemisches höher als bei einem nicht vorbehandelten Gemisch. Weiterhin weist das Beispiel 1 deutlich höhere Nickelwiederfindungsraten als die Vergleichsbeispiele auf. Die Selektivität bzgl. Butadien wird im erfindungsgemäßen Verfahren um etwa 1% gegenüber der Referenz erhöht. In Beispiel 1 wurde weniger Butadien (Überschußkomponente) eingesetzt, als bei den Vergleichsbeispielen 1 und 2. Dennoch ist Umsatz an HCN (Unterschußkomponente) höher. Dieser Effekt ist überraschend, da ansonsten mit zunehmender Menge an Überschußkomponente auch der Umsatz an Unterschußkomponente zunehmen sollte. Auch im Vergleichsbeispiel 2 mit deutlich höherem Katalysatoranteil werden die Ergebnisse von Beispiel 1 nicht erreicht.

### Beispiel 2

### semikontinuierliche Hydrocyanierung von teilhydriertem C₄-Schnitt

40 g Toluol,
20,3 g vorhydrierter C₄-Schnitt (= 7,9 g 1,3-Butadien),
4,7 g Katalysatorzusammensetzung, enthaltend:
   25 Gew.-% Tetrakis(tri-m/p-tolylphosphit)Ni(0)
   60 Gew.-% Tri-m/p-tolylphosphit
   15 Gew.-% 3-Pentennitril,
werden in einen mit Glas ausgekleideten Autoklaven gefüllt und 2 h auf 90°C erhitzt. Währenddessen werden 3,2 g HCN und 40 g Toluol zudosiert. Anschließend läßt man 2 h bei 90°C nachreagieren.
Umsatz (nach Maßanalyse der nicht umgesetzten HCN): 98,0%
Ausbeute (nach Integration des Gaschromatogramms): 98,8% bzgl. HCN

### Beispiel 3:

### Semikontinuierliche Hydrocyanierung von teilhydriertem C₄-Schnitt mit Tri(m/p-tolyl)phosphit

In einem Glasautoklaven werden bei Raumtemperatur 20,3 g teilhydrierter C₄-Schnitt (entsprechend 7,9 g (0,14 mol) 1,3-Butadien), 2,75 g einer Katalysatormischung (Zusammensetzung: 0,69 g (0,468 mmol) Tetrakis(tri-m/p-tolylphosphit)nickel(0), 1,65 g (4,68 mmol) Tri-m/p-tolylphosphit und 0,41 g 3-Pentennitril) und 40 g Toluol vorgelegt und auf 90 °C erhitzt, wobei sich ein Anfangsdruck von 4,4 bar einstellt. Über einen Zeitraum von 100 Minuten wird eine Mischung aus 3,2 g (0,117 mol) frisch destillierter Blausäure in 40 g Toluol kontinuierlich zudosiert. Danach ist der Druck auf 3,1 bar gefallen. Anschließend lässt man zum Vervollständigen der Reaktion noch 120 Minuten bei etwa 90 °C nachreagieren. Der Verlauf der Reaktion wird über Druck- und Temperaturmessung verfolgt. Nach dem Abkühlen wird der flüssige Reaktionsaustrag analysiert.

Umsatz (nach Maßanalyse der nicht umgesetzten HCN): 97,6%

GC-Analytik (Säule: 30 m Stabil-Wachs, Temperaturprogramm: 5 Minuten isotherm bei 50 °C, danach Aufheizen mit einer Geschwindigkeit von 5 °C/min auf 240 °C, Gaschromatograh: Hewlett Packard HP-5890) mit internem Standard (Benzonitril): 96,8 % 3-Pentennitril und 2-Methyl-3-butennitril, bezogen auf eingesetzten Cyanwasserstoff.

Verhältnis 3-Pentennitril : 2-Methyl-3-butennitril = 1,64:1.

### Beispiel 4:

### Semikontinuierliche Hydrocyanierung von teilhydriertem C₄-Schnitt unter Einsatz von Ligand A

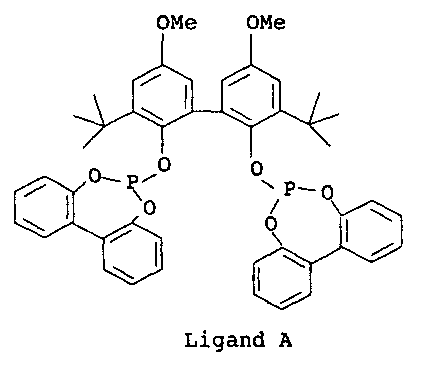

Die Herstellung des eingesetzten Liganden A ist in der WO 95/14 659 und der dort zitierten Literatur beschrieben.

In einem Glasautoklaven werden bei Raumtemperatur 0,41 g (1,5 mmol) Bis(1,5-cyclooctadien)nickel(0), 0,44 g Ligand A und 10 g Toluol miteinander vermengt. Danach wird eine Mischung aus 20,8 g teilhydriertem C₄-Schnitt (entsprechend 8,1 g (0,15 mol) 1,3-Butadien)in 40 g Toluol zugegeben. Der Glasautoklave wird fest verschlossen und die Reaktionsmischung auf 80 °C erhitzt, wobei sich ein Anfangsdruck von 3,1 bar einstellt. Über einen Zeitraum von 120 Minuten wird eine Mischung aus 4,0 g (0,15 mol) frisch destillierter Blausäure in 40 g Toluol kontinuierlich zudosiert. Danach ist der Druck auf 1,5 bar gefallen. Anschließend lässt man zum Vervollständigen der Reaktion noch 120 Minuten bei etwa 80 °C nachreagieren. Der Verlauf der Reaktion wird über Druck- und Temperaturmessung verfolgt. Nach dem Abkühlen wird der flüssige Reaktionsaustrag analysiert.

Umsatz (nach Maßanalyse der nicht umgesetzten HCN): 88,7%

GC-Analytik (Säule: 30 m Stabil-Wachs, Temperaturprogramm: 5 Minuten isotherm bei 50 °C, danach Aufheizen mit einer Geschwindigkeit von 5 °C/min auf 240 °C, Gaschromatograph: Hewlett Packard HP-5890) mit internem Standard (Benzonitril): 89,3 % 3-Pentennitril und 2-Methyl-3-butennitril, bezogen auf eingesetzten Cyanwasserstoff.

Verhältnis 3-Pentennitril : 2-Methyl-3-butennitril = 0,27:1.

### Beispiel 5:

### Semikontinuierliche Hydrocyanierung von teilhydriertem C₄-Schnitt unter Einsatz von Ligand B

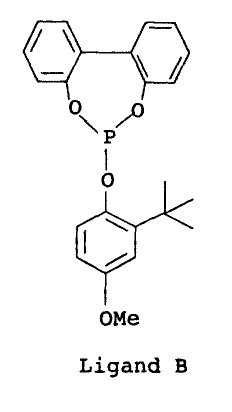

Die Herstellung des Liganden B ist in der WO 95/29 153 beschrieben.

In einem Glasautoklaven werden bei Raumtemperatur 0,41 g (1,5 mmol) Bis(1,5-cyclooctadien)nickel(0), 2,36 g (6 mmol) Ligand B und 10 g Toluol miteinander vermengt. Danach wird eine Mischung aus 20,8 g teilhydriertem C₄-Schnitt (entsprechend 8,1 g (0,15 mol) 1,3-Butadien) in 40 g Toluol zugegeben. Der Glasautoklave wird fest verschlossen und die Reaktionsmischung auf 80 °C erhitzt, wobei sich ein Anfangsdruck von 3,1 bar einstellt. Über einen Zeitraum von 100 Minuten wird eine Mischung aus 4,0 g (0,15 mol) frisch destillierter Blausäure in 40 g Toluol kontinuierlich zudosiert. Danach ist der Druck auf 2,1 bar gefallen. Anschließend lässt man zum Vervollständigen der Reaktion noch 140 Minuten bei etwa 80 °C nachreagieren. Der Verlauf der Reaktion wird über Druck- und Temperaturmessung verfolgt. Nach dem Abkühlen wird der flüssige Reaktionsaustrag analysiert.

Umsatz (nach Maßanalyse der nicht umgesetzten HCN): 81,3%

GC-Analytik (Säule: 30 m Stabil-Wachs, Temperaturprogramm: 5 Minuten isotherm bei 50 °C, danach Aufheizen mit einer Geschwindigkeit von 5 °C/min auf 240 °C, Gaschromatograph: Hewlett Packard HP-5890) mit internem Standard (Benzonitril): 81,9 % 3-Pentennitril und 2-Methyl-3-butennitril, bezogen auf eingesetzten Cyanwasserstoff.

Verhältnis 3-Pentennitril : 2-Methyl-3-butennitril = 1,24:1.

Die Unterschiede zwischen den mittels Maßanalyse und den mittels Gaschromatographie ermittelten Umsätzen liegen im Rahmen der Messgenauigkeit der beiden Methoden.

## Patentansprüche

1. Verfahren zur Herstellung von Gemischen monoolefinischer C₅-Mononitrile mit nichtkonjugierter C=C- und C ≡ N-Bindung durch katalytische Hydrocyanierung eines 1,3-Butadien-haltigen Kohlenwasserstoffgemisches, **dadurch gekennzeichnet, daß** man das Kohlenwasserstoffgemisch mit Wasserstoff in Gegenwart eines Hydrierungskatalysators umsetzt, der befähigt ist, Alkine und 1,2-Diene mit hoher Selektivität zu hydrieren, ohne den Gehalt an 1,3-Butadien wesentlich zu verringern, um aus dem Kohlenwasserstoffgemisch Alkine, 1,2-Diene und Gemische davon teilweise oder vollständig zu entfernen, so dass der Anteil derjenigen Komponenten, welche die katalytische Hydrocyanierung beeinträchtigen, verringert wird, und man das so erhaltene Gemisch anschließend katalytisch hydrocyaniert.

2. Verfahren nach Anspruch 1, **dadurch gekennzeichnet, daß** man ein Kohlenwasserstoffgemisch mit einem 1,3-Butadiengehalt von mindestens 10 Vol.-%, bevorzugt mindestens 25 Vol.-%, insbesondere mindestens 40 Vol.-%, einsetzt.

3. Verfahren nach einem der vorhergehenden Ansprüche, **dadurch gekennzeichnet, daß** man als 1,3-Butadien-haltiges Kohlenwasserstoffgemisch einen C₄-Schnitt aus der Erdölverarbeitung einsetzt.

4. Verfahren nach einem der vorhergehenden Ansprüche, **dadurch gekennzeichnet, daß** man ein Kohlenwasserstoffgemisch einsetzt, das 10 bis 50 Vol.-%, bevorzugt 25 bis 47 Vol.-% 1,3-Butadien sowie zusammen etwa 0,1 bis 5, bevorzugt 0,2 bis 2,5 Vol.-% Alkine und/oder 1,2-Diene umfaßt.

5. Verfahren nach einem der vorhergehenden Ansprüche, **dadurch gekennzeichnet, daß** der zur Hydrocyanierung eingesetzte Katalysator zusätzlich zur Cyanwasserstoffaddition auch die Stellungs- und Doppelbindungsisomerisierung des Kohlenwasserstoffgemisches und/oder der Nitrile katalysiert.

6. Verfahren nach einem der vorhergehenden Ansprüche, **dadurch gekennzeichnet, daß** man ein Produktgemisch erhält, welches isomere Pentennitrile und Methylbutennitrile, wie 3-Pentennitril, 2-Pentennitril, 4-Pentennitril, 2-Methyl-2-butennitril, 2-Methyl-3-butennitril umfasst.

7. Verfahren zur Herstellung von Adipodinitril, **dadurch gekennzeichnet, daß** man ein gemäß den Ansprüchen 1 bis 6 hergestelltes Gemisch von C₅-Mononitrilen, gegebenenfalls nach weiterer Aufarbeitung oder Isomerisierung katalytisch hydrocyaniert.

## Claims

1. A process for preparing mixtures of monoolefinic C₅ mononitriles having nonconjugated C=C- and C ≡ N bonding by catalytic hydrocyanation of a hydrocarbonaceous mixture containing 1,3-butadiene, which comprises reacting the hydrocarbonaceous mixture with hydrogen in the presence of a hydrogenation catalyst capable of hydrogenating alkynes and 1,2-dienes with high selectivity without significantly diminishing the 1,3-butadiene content in order to partially or completely remove alkynes, 1,2-dienes and mixtures thereof from the hydrocarbonaceous mixture so that the proportion of those components which impair the catalytic hydrocyanation is diminished and then subjecting the resulting mixture to catalytic hydrocyanation.

2. A process as claimed in claim 1, wherein the hydrocarbonaceous mixture used has a 1,3-butadiene content of at least 10 % by volume, preferably at least 25 % by volume, in particular at least 40 % by volume.

3. A process as claimed in any of the preceding claims, wherein the 1,3-butadiene-containing hydrocarbon mixture used is a C₄ cut from petroleum processing.

4. A process as claimed in any of the preceding claims, wherein the hydrocarbon mixture used comprises from 10 to 50 % by volume, preferably from 25 to 47 % by volume, of 1,3-butadiene and also in total from about 0.1 to 5, preferably from 0.2 to 2.5, % by volume of alkynes and/or 1,2-dienes.

5. A process as claimed in any of the preceding claims, wherein the catalyst used for the hydrocyanation catalyzes the position and double bond isomerization of the hydrocarbonaceous mixture and/or of the nitriles as well as the hydrogen cyanide addition.

6. A process as claimed in any of the preceding claims, wherein the product mixture obtained comprises isomeric pentenenitriles and methylbutenenitriles, such as 3-pentenenitrile, 2-pentenenitrile, 4-pentenenitrile, 2-methyl-2-butenenitrile and 2-methyl-3-butenenitrile.

7. A process for preparing adiponitrile, which comprises catalytically hydrocyanating a C₅ mononitrile mixture prepared as claimed in any of claims 1 to 6, optionally after further workup or isomerization.

## Revendications

1. Procédé de préparation de mélanges de C₅-mononitriles monooléfiniques présentant une liaison C=C et C≡N non conjuguée par hydrocyanation catalytique d'un mélange hydrocarboné contenant du 1,3-butadiène, **caractérisé en ce qu'**on fait réagir le mélange hydrocarboné avec de l'hydrogène en présence d'un catalyseur d'hydrogénation qui est capable d'hydrogéner avec une sélectivité élevée des alcynes et des 1,2-diènes, sans réduire sensiblement la teneur en 1,3-butadiène, pour éliminer partiellement ou totalement du mélange hydrocarboné des alcynes, des 1,2-diènes et leurs mélanges, de façon à réduire la fraction de ces composants qui influencent désavantageusement l'hydrocyanation catalytique, et **en ce qu'**on hydrocyane ensuite par voie catalytique le mélange ainsi obtenu.

2. Procédé suivant la revendication 1, **caractérisé en ce qu'**on met en oeuvre un mélange hydrocarboné présentant une teneur en 1,3-butadiène d'au moins 10% en volume, de préférence d'au moins 25% en volume, en particulier d'au moins 40% en volume.

3. Procédé suivant l'une des revendications précédentes, **caractérisé en ce qu'**on met en oeuvre, comme mélange hydrocarboné contenant du 1,3-butadiène, une coupe C₄ provenant du traitement de pétrole.

4. Procédé suivant l'une des revendications précédentes, **caractérisé en ce qu'**on met en oeuvre un mélange hydrocarboné qui comporte 10 à 50% en volume, de préférence 25 à 47% en volume, de 1,3-butadiène ainsi que conjointement environ 0,1 à 5, de préférence 0,2 à 2,5, % en volume d'alcynes et/ou de 1,2-diènes.

5. Procédé suivant l'une des revendications précédentes, **caractérisé en ce que** le catalyseur mis en oeuvre pour l'hydrocyanation catalyse, en plus de l'addition d'acide cyanhydrique, l'isomérisation de position et de double liaison du mélange hydrocarboné et/ou des nitriles.

6. Procédé suivant l'une des revendications précédentes, **caractérisé en ce que** qu'on obtient un mélange produit qui comporte des méthylbutènenitriles et des pentènenitriles isomères, comme du 3-pentènenitrile, du 2-pentènenitrile, du 4-pentènenitrile, du 2-méthyl-2-butènenitrile, du 2-méthyl-3-butènenitrile.

7. Procédé de préparation d'adipodinitrile, **caractérisé en ce qu'**on hydrocyane par voie catalytique un mélange de C₅-mononitriles préparés suivant l'une des revendications 1 à 6, éventuellement après un traitement ultérieur ou une isomérisation.
